# EUROPEAN PATENT APPLICATION

(11) **EP 2 184 084 A1**
(43) Date of publication of application: **12.05.2010**
(21) Application number: 07788675.2
(22) Date of filing: 16.07.2007
(51) Int. Cl.: A61N 1/36, A61N 1/06

(54) **DEVICE FOR NEURONAL THERAPIES**

(71) Applicant: Arriaza Muñoz, Francisco José, 08203 Sabadell, Barcelona (ES)
(72) Inventor: Arriaza Muñoz, Francisco José, 08203 Sabadell, Barcelona (ES)
(74) Representative: Isern-Jara, Nuria
(86) International application number: PCT/ES2007/000430
(87) International publication number: WO 2009/010596

(57) **Abstract**

Device comprising a high frequency and/or very high frequency generator (1) functioning by means of coils (11), and associated with two main electrodes (2), which are respectively configured by a core (21) of insulating material with the front end (22) in point form and surrounded by a flexible insulating tubular body (23) extended on the point end (22) of the electrode for separate and safe positioning thereof with respect to the eyes of the patient on which said electrodes (2) are placed in a use operation, in order to cause stimulation of the nervous system and improvement of neuronal transmission by means of the circulation of high frequency currents. Both electrodes (2) are associated with at least one element for support (3) and positioning over the eyes of the patient, based on an element for fastening (4) to the head or a cabin (6).

## Description

### Object of the invention

The present invention relates to a device for neuronal therapies, mainly applicable to the body of human beings, for the stimulation of the nervous system and for enhancing neuronal transmission.

### Background of the invention

There is a great diversity of therapies which are applicable to the body of the human being or to the body of other living beings for the stimulation of its tissues and organs with a therapeutic aim or simply for tonifying purposes.

An example of these therapies is the rippled sinmerization, wherein a high frequency and/or very high frequency generator functioning by means of coils is used, which is associated to one or more electrodes for its application on the body of the patient, thus allowing passage of low currents of said frequencies through the body tissues of said patient, obtaining a substantial stimulation effect.

However, for the application of this therapy the electrode is mainly metallic, which presents multiple problems for concentrating the therapy on certain parts of the body.

### Summary of the invention

The device for neuronal therapies, object of this invention, presents several particular technical features aimed at causing stimulation of the nervous system in a substantially direct way and for achieving improved neuronal transmission in diseases related to an optimal functioning of said nervous system, such as fibromyalgia and others.

In fact, the device comprises two electrodes which are respectively configured by a core of insulating material with the front end of pointed form, this core being surrounded by a flexible tubular insulating body, such as a foam, extended on the pointed end of the electrode for separate and safe positioning thereof with respect to the eyes of the patient over which said electrodes are placed in a use operation, in order to cause stimulation of the nervous system and improvement of neuronal transmission.

After the front eyepieces of the flexible tubular body have been placed on the eyes, the end of each electrode core remains a short distance from them, although not entering in contact with them, allowing to perform a circulation of high frequency and/or very high frequency currents directly through the eyes and the optic nerves, reaching the brain in a very direct and efficacious way.

Both electrodes are associated with at least one element for support and positioning over the patient in a safe and comfortable way, given their delicate positioning.

The insulating core of the electrode is made of wood or other insulating material, reducing thereby the possible uneasiness provoked in the patient when using a metallic electrode which is placed in front of the eyes. It also produces a milder and less disturbing discharge effect compared to the use of a metallic electrode, thus preventing any unpleasent feeling for the patient.

In a first embodiment, the charge coil is situated in the electrode, sending a low frequency current from the generator for the multiplication thereof. In a second embodiment, the charge coil is situated in the generator itself, transmitting the high frequency current directly to the core of the electrode.

In a basic embodiment, the support of the electrodes comprises an element for fastening to the head of the user, such as a helmet or harness, and at least one adjustable positioning arm for approaching or removing the electrodes, in order to achieve a correct positioning over the eyes and a stable support.

In a more complete and detailed embodiment, the support of the electrodes is associated to a therapy cabin, which has a support surface for the patient, preferably in a lying position, said surface having three additional electrodes in contact with the body to improve channeling of the high frequency and/or very high frequency currents through the brain, the spine and down to the feet. Therefore, these additional electrodes are positioned in positions corresponding to head, body and feet. These additional electrodes are preferably of wood or other insulating material, as are the cores of the main electrodes.

The cabin comprises a top lid for partial closure, preferably covering the body and leaving the head exposed, for the positioning of the support of the main electrodes in front of the face of the user, said electrodes also being anchored on said lid.

Of the additional electrodes present in the cabin, the electrode for contacting the feet is positioned on a longitudinally movable support for adjustment to the height of the user. This is because the position of the patient inside the cabin is dictated by the position of the head of the patient in relation to the main electrodes.

This additional electrode for the feet is configured by two L-shaped plates to help support the bottom and heel areas of the feet of the user.

In an alternative embodiment, the cabin includes a subsonic (non-audible) vibration device comprising a low-frequency signal generator and one or more loudspeakers housed in the bottom portion of said cabin. This subsonic vibration provides advantages of synergy when operating together with the high frequency and/or very high frequency currents therapy produced by the main and additional electrodes. In addition, the cabin functions as ressonance cabin of the subsonic vibrations, enhancing their beneficial effects on the body of the patient by virtue of the substantial cellular vibrations which are produced.

The cabin can be provided with a climatizer in order to adjust the temperature during the application of the treatment to a cold, warm or ambient temperature.

### Description of the figures

To complement the description herein provided, and with an aim to help the understanding of the features of the invention, a set of drawings is attached herein, in which, by way of illustration and not limitation, the following is shown:

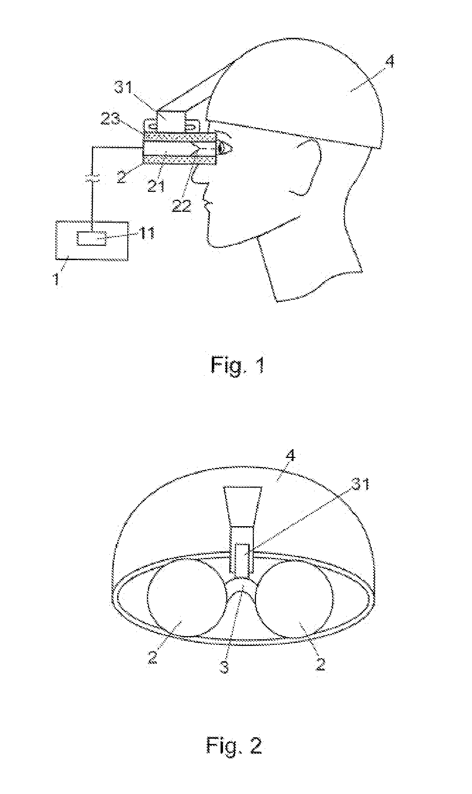

Figure 1 shows a side view of the device in portable configuration on the head of a patient.
Figure 2 shows a front view of the device of the above figure.

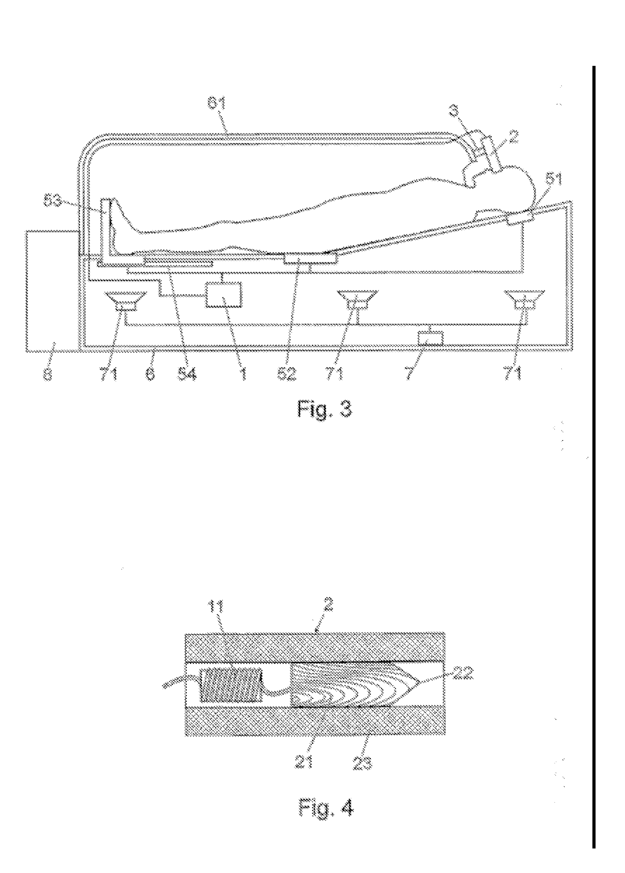

Figure 3 shows a side view in lengthwise section of the cabin device in complete configuration.
Figure 4 shows a lengthwise section of an alternative embodiment of an electrode with the charge coil next to the wooden core.

### Preferred embodiment of the invention

As can be seen in the referred figures, the device basically comprises a high frequency and/or very high frequency generator (1) functioning by means of a coil (11), associated to two main electrodes (2) positioned in front of the eyes of the user. Each electrode (2) comprises an elongated core (21) made of wood or other insulating material with one end (22) configured in pointed form, said core (21) being connected on its rear portion to the generator (1) by means of a connection cable, and said core (21) being surrounded by a flexible insulating tubular body (23) made of foam, which covers it and extends beyond the front pointed end (22) of the core (21) for protecting and separating it from the mouth by a small distance.

In a first portable embodiment, the two electrodes (2) are joined by a support element (3) and an adjustable telescopic positioning arm (31) to a fastening element (4) held onto the head, configured as a helmet, which positions the electrodes (2) in a front position oriented towards the face of the user, at the height of the eyes, allowing to approach or remove said electrodes to the eyes as necessary.

In a more complete embodiment, the device comprises one or more additional electrodes (51, 52, 53) connected to the generator (1). These electrodes (51, 52, 53) are comprised of plaques of insulating material, in the present case of wood, for their application to the head, body and/or feet of the patient. These electrodes (51, 52, 53) are positioned on a support surface for the patient, mainly in a lying position, in a therapy cabin (6), which has a lid (61) partially covering the body up to the face, which is left uncovered, the electrodes (2) being positioned in this upper portion of the lid (61), on a position-adjustable support element (3), the high frequency and/or very high frequency generator being in the lower portion of the cabin (6).

The additional electrode (53) for the feet comprises two plates joined together forming an "L" and is positioned on a longitudinally movable support (54) for adjustment to the height of the user.

The cabin (6) comprises, inside thereof, a subsonic (non-audible) vibration device comprising a low-frequency signal generator (7) and one or more loudspeakers (71) housed in its bottom portion, for the vibrations produced to ressonate with the cabin cavity (6), causing cellular vibrations in the patient. The cabin (6) also has an adjacent climatizer (8) for the inside thereof.

Having described sufficiently the nature of the invention, together with an example of a preferred embodiment, it is stated for such purposes as may arise that the materials, form, size and arrangement of the described elements can be modified without altering essential features of the invention as claimed below.

## Claims

1. Device for neuronal therapies, of the type comprising a high frequency and/or very high frequency generator (1) functioning by means of coils (11), associated to one or more electrodes, **characterized in that** it comprises two electrodes (2), comprising respectively a core (21) of insulating material with a front end (22) of pointed form and surrounded by a flexible insulating tubular body (23), extended on the pointed end (22) of the electrode for separate and safe positioning thereof with respect to the eyes of the patient, on which said electrodes (2) are placed in a use operation in order to cause stimulation of the nervous system and improvement of neuronal transmission by means of the circulation of high frequency and/or very high frequency currents, and **in that** both electrodes (2) are associated to at least one element (3) for support and positioning over the eyes of the patient.

2. Device according to claim 1, **characterized in that** the core (21) of the electrode (2) is of wood or of another insulating material.

3. Device according to claim 1, **characterized in that** the charge coil (11) is housed inside the electrode (2).

4. Device according to claim 1, **characterized in that** the charge coil (11) is housed inside the generator (1).

5. Device according to claim 1, **characterized in that** the support element (3) of the electrodes (2) comprises an element (4) fastening on the head of the user and at least one adjustable positioning arm for approaching or removing the electrodes (2) with respect to said fastening element (4).

6. Device according to claim 1, **characterized in that** the generator (1) is associated to one or more additional electrodes (51, 52, 53), comprising plates of insulating material operationally applicable to head, body and/or feet of the patient.

7. Device according to claim 6, **characterized in that** the additional electrodes (51, 52, 53) are of wood or of another insulating material.

8. Device according to claim 6, **characterized in that** the additional electrodes (51, 52, 53) are arranged on a support surface for the patient, preferably in a lying position, inside a therapy cabin (6), and on which the support element (3) of the main electrodes (2) is also secured on a position above.

9. Device according to claim 8, **characterized in that** the cabin (6) comprises a lid (61) for partial top closure.

10. Device according to claim 8, **characterized in that** the electrode (53) is arranged on a longitudinally movable support (54) for adjustment to the height of the user.

11. Device according to claim 10, **characterized in that** the electrode (53) is configured by two "L-shaped" plates to help support the bottom and heel areas of the feet of the user

12. Device according to claim 8, **characterized in that** the cabin (6) comprises a subsonic vibration device comprising a generator (7) of low-frequency signal and one or more loudspeakers (71) housed in the bottom portion of said cabin.

13. Device according to claim 8, **characterized in that** the cabin (6) has a climatizer (8).
